# EUROPEAN PATENT SPECIFICATION

(11) **EP 1 299 146 B1**
(45) Date of publication and mention of the grant of the patent: **27.10.2004**
(21) Application number: 01951103.9
(22) Date of filing: 03.07.2001
(51) Int. Cl.: A61M 29/02, A61M 25/10, A61M 5/142, F41B 9/00

(54) **INSTRUMENT FOR FLUID INJECTION AND DILATATION PROBE FOR IMPLANTATION IN BODY CAVITIES**
VORRICHTUNG ZUM EINSPRITZEN EINES FLUIDS UND DILATIONSSONDE ZUM IMPLANTIEREN IN KÖRPERHÖHLEN
INSTRUMENT D'INJECTION DE FLUIDE ET SONDE A DILATATION DESTINEE A UNE IMPLANTATION DANS DES CAVITES CORPORELLES

(30) Priority: 04.07.2000 YU 42400
(43) Date of publication of application: 09.04.2003
(73) Proprietor: Arsenijevic, Slobodan, 34000 Kragujevac (YU); Cakic, Nenad, 11000 Beograd (YU)
(72) Inventor: Arsenijevic, Slobodan, 34000 Kragujevac (YU); Cakic, Nenad, 11000 Beograd (YU)
(74) Representative: Eder, Christian, Dipl.-Ing.
(86) International application number: PCT/YU2001/000016
(87) International publication number: WO 2002/002175

(56) References cited:
- WO-A-94/02195
- US-A- 2 862 497
- US-A- 3 055 371
- US-A- 4 332 254
- US-A- 5 176 647
- US-A- 5 184 755
- US-A- 5 254 092
- US-A- 5 352 210
- US-A- 5 993 402

## Description

### The field of technique to which the invention applies

The invention, in general, appertains to a field of current needs of life, that is, in a field of health, care and entertainment, and, concretely, in a field of devices for medicine implantation or application or devices for trans-duction of the body substances. The invention could, in general terms, secondarily appertain in a field of armament and mining, that is the domain of projectile launching arms without the usage of explosive charge.

According to the International invention classification, the object of invention is designated with basic classification symbols: A 61 M 5/145, involving devices for filling or cleaning with usage of pressurized reservoirs, for instance by means of pistons, A 61 M 29/04, involving dilatators that can be inflated, made of materials that can be blown up, and F 41 B 9/00 involving water pistols, that is liquid ejection pistols.

### The technical problem

The technical problem to be solved by this invention is following: how to design an instrument capable of filling with fluid, most favorably distilled water, of the dilative probe for implanting in human body cavities. This device should be simply designed, practical and safe for human usage. It should also be suitable for industrial manufacturing, as well as sterilization and maintenance. The problem of the probe, suitable for industrial manufacturing, and absolutely safe from rupture and tissue damage, is also solved. This way the problem of designing a probe for a gullet dilatation enabling a patient to be fed during the treatment according to the surgeon request is also solved. Hereby the complete device, consisting of an instrument and a probe, for even and safe probe dilation by means of fluid injection in a cylinder shaped probe would be made available. Sterilized probes are for single usage only.

### The state of the art

In non-patent and patent literature there is no knowledge of the solution for the complete device that could be used by surgeon to do a controlled fluid injection in a dilative probe enlarging it, but a number of different designs is well known, especially for the dilating probes for implantation in body cavities, that is opening - widening of the human cavities during an rectal, cervical angiological plastic surgeries.

There are a lot of reasons in current medical practice that make dilatation of the human cavities, especially a cervical canal, a necessity. As a result, a variety of methods to achieve that exist. However, there are a lot of unwanted side effects, both medical and physical, because earlier devices did not comprehend the advantages of this invention.

It was a general practice, in early means of human uterus dilatation, to put in a set of inserts with successively increasing diameters so the dilatation would be achieved. Overcoming of the unwanted side effects was attained with dilatators for body canal opening by means of application of radial force. There are a number of solutions that made radial widening of the canals possible and this was solved on a laboratory level with usage of materials that are not to be in contact with human tissue, but the problem of patient protection from probe bursting, as well as industrial manufacturing problem remained. Additionally, previous methods also comprised the use of absorbing material to be initially inserted in a cervix to dilate it after sucking up body (or some other) fluids. Prior practice have also known the ways to dilate the cervix by means of inflating devices, which had different complications in usage and are surpassed by this invention.

In accordance with previously mentioned and existing state of the art, some of the mentioned patent specifications present the current technical solutions: claimed by Robert Irvine Leninger in U.S. Pat. No. 3,900,033 A, named DILATOR FOR THE CERVICAL CANAL; claimed by Richard F. Kronser in U.S. Pat. No. 4,089,337 A, named UTERUS CATHETER AND MANIPULATOR WITH INFLATING SEAL; claim by Stanley B. Levy U.S. Pat. No. 4,490,421 A named BALLOON AND IT'S MANUFACTURING; claim by Gao Yang no. CN 2352198U named DISPOSABLE CERVICAL DILATOR and claim by Jiang Shan No. CN 1088459 named INTELLIGENT AUTOMATIC WOMB DILATOR.

When we speak about concrete solutions, British Patent GB 2 297 036 A to Alexander KOSTIC «Gynaecological pneumatic Parachute Dilator» shows and describes the inflating cervical dilator which consists of an inner balloon, mounted on a flexible steel guide, covered with a sleeve made of wrapped silk which is covered by the outer balloon. With the inflation of the internal balloon, the silk sleeve slides out of the plastic body, so it could take cylindrical shape suitable for cervical dilatation. Protective outer balloon takes the same shape and protects for leaks. When the silk sleeve is once inflated, it cannot be inserted again, so the device is for single usage only. The third, shortened piece of balloon holds the lower part of the silk sleeve and is also used for pressure testing of the system during manufacture. Evacuation holes are made in the plastic body so the air could be evacuated in case of emergency.

Another patent, No. US 4,664,114 A named "Cervical Canal Dilator" refers to a new design of a device used for dilating various body cavities, especially a human cervix. The device consists of a hollow cylindrical unit, suitable for insertion in a human uterus through a cervix, covered with an inflatable diaphragm that could be selectively swollen while the device is inside the uterus, thus preventing the unwanted extraction. Additionally, when a device is already inserted through a cervix, with a diaphragm, positioned around the outer part of the device placed in a uterus cavity, already inflated, inflation of the second diaphragm, positioned around the outer part of the device near the cervical wall, occurs, thus inducing the selective dilatation of the cervix as a result of a selective inflation of a second diaphragm.

Patent No. US 3,900,033 A named "Cervical Canal Dilator", describes an inflatable device aimed for dilatation of various body cavities, especially human cervix, consisting of a wrapping with an enlarged swollen part on one end and a shield at the opposite end. The wrapping can be inflated with suitable gases as carbon dioxide or fluids like physiological solution - salty distilled water or similar.

Patent No. US 3,848,602 A named "Abortion Facilitating Device and Process" shows a process of cervical dilatation preceding the abortion and consisting of steps during which the abortion device is inserted through cervix. Then, the dilator part next to cervix is, gradually and uniformly, radially expanding in uterus cavity with a controlled rate so the dilatation of cervix could be achieved by liquid injection. When the cervix is dilated fluid is removed, so the abortion could be finished after the extraction of the dilator. The instrument for cervical dilatation consists of a tube member with three extending ducts: one duct ends substantially in a distal region and communicates with a dilation part, which takes cylindrical shape after widening; second duct ends distally from first duct in an extensible hardening member; and the third duct ending at the tip of the mentioned tube member, so the flow of the fluid in and out of the uterus cavity is enabled.

In another patent, US 4,832,691 A named "Pneumatic Bougie, particularly for treatment of stenoses", shows a rubber tube with a balloon shaped dilator slipped over the gastroscope. The balloon dilator comprises an inflatable balloon whose diameter is limited to a given upper value during dilation. The balloon is made of homogeneous flexible, elastic material. It has a shape of a truncated cone. A flexible tube of a given length goes through a balloon, which is attached to this tube. The dimensions of the tube are chosen in a way so the gastroscope could get through it. The transfer tube is opened inside the balloon to enable the inflation. The transfer tube is connected to the blood pressure gauge, which has a blood vent on it. Replacing the rubber tube enables conical balloons of various diameters to be inserted in shrunken stomach - digestive parts of the body. With the inflation of the balloon, the shrunken element will be dilated to a predefined diameter, enabling the passage of a partly digested food.

Patent No. JP10305042 named "Cervical Canal Dilator" solves a problem of providing the cervical canal dilator, which would not tire a doctor even in cases of prolonged operations and which would not require special skills for a manual cervical canal dilating method. The problem is solved by means of a dilator with the inserting member; having a tip to be put in a vagina, with an extension attached to the member tip and stimulating parts for inserting in a cervical cannel, and guides attached to a base member tip, which guides the member.

Patent No. CN 1088459 named "Intelligent Automatic Uterus Dilator" shows a dilator consisting of a handle, uterus probe, fluid leading system, box for fluid injection and intelligent control panel. The mentioned handle is coated with a nontoxic transparent material with a front end connected with the probe. Both handle and probe are hollow and joined in an integrated whole. The outside of the probe is covered with elastic film, tightly terminated with a cap. From the intelligent control panel, the fluid injection box and the fluid leading system, the film in the cervix could automatically dilate up to an immediate diameter. Thus a doctor operates comfortably and easily, while a patient feels less pain, and tissue irritation, bleeding and infection are avoided.

A dilator with the features of the preamble of claim 1 is disclosed in US 4,332,254 A.

Although the present state of the art knows a variety of techniques and devices, the present invention applies to a new design of the instrument for fluid injection and a dilator, which is to be used as a dilator for a variety of body cavities, especially human cervix. This device has different advantages in regard to the mentioned devices not mentioned in a present state of the art.

Therefore, the objective of the invention is to design a new device, which is to be used as an instrument with a dilator for various body cavities and surpass certain problems and disadvantages of the devices known in a present state of the art.

### Short description of Invention essence

Invention refers to an instrument for fluid injection with an inserted dilatation probe for implanting in human cavities. The instrument basically consists of a handle, filling valve, relief-adjusting valve, intake valve, thrust valve, cylindrical extension and a coupling for a dilating probe attachment. A "fluid" needed for the operation of the instrument assumes distilled water as the most favorable solution. The intake of the coupling for the dilatation probe attachment is opened with an especial cover during the sterilization. It is important to emphasize that the dilatation probes are provided for single-use.

The instrument consists of a handle enclosed by a cover at the base. At the inside of the cover and in the handle cavity the feed valve is attached, used for priming the instrument with the distilled water necessary for it's operation. Besides the feed valve, in the handle cavity, the cylinder of a variable volume is located, attached to a handle cover by means of a threaded joint. A movable piston sealed with an "O" ring is located in a cylinder.

The relief-adjusting valve is located in the handle, and, with it's base, is in connection with the handle cavity. Besides, the intake valve is located along the axis of the handle body, and is in connection, through the intake tube, with the handle cavity and the trigger piston. At the upper part and along the intake valve axis the thrust valve is located. It is in connection with the central canal, which is located in the upper part and goes through the handle and the cylindrical extension. The instrument handle is connected with the coupling for a dilatation probe attachment through the cylindrical extension.

Dilatation probe for body cavities implantation consists of a fitting, which is inserted in the instrument coupling. At the inside of the probe, and at the extension of the fitting, there is a space limited by the inner elastic diaphragm and the armored fabric made of polyethylene with directed molecular chains wrapped in a paper wrapping. All this is shrouded with the probe cover, which presses the end of the inner elastic diaphragm against the cannel in the probe fitting. Outer elastic diaphragm, which holds the injected fluid, and is attached to the probe cover cannel, is placed over the whole package.

The dilatation probe, as a production variant, aimed for gullet dilatation, consists of the instrument fitting, with the nonreturn valve at the inlet. The hose with openings for feeding is located along the hose for the pressurized fluid, which is wrapped with the outer diaphragm, armored fabric and the inner diaphragm. The operation of the probe is achieved by implanting the dilatation probe through a nose until the dilating part reaches the place where the stenosys is. When this is done, the pumping of the fluid with the instrument begins, causing the dilatation. The dilatation is performed through the nonreturn valve until the diameter requested by the surgeon is achieved. After the dilatation is finished, the instrument is detached and the probe stays at the position for the time needed for the tissue accommodation. The nonreturn valve maintains the pressure in the probe. After that, the instrument is attached again for further dilatation. This procedure is repeated until the end of dilatation. When the dilatation is finished, the hose for the pressurized fluid is cut and the probe is pulled out of the gullet. The probe is made of thermolabile material and is for a single usage only, so the sterilization is performed in a packed form with a cobalt bomb.

The instrument for a fluid filling and dilatation probes for implantation in body cavities, according to this invention, have several advantages, some of the most important are specified as:
- Absolute safety of the patient, concerning the procedure as well as the medical hygienic rules
- The possibility for the industrial manufacturing
- Simple and safe design
- The work with the probe is simple and practical
- Long instrument lifetime

### Short description of drawings

For the purpose of easier understanding of the invention, as well as the description of the usage in practice, applicant is referring to the enclosed drawings, where:
Figure 1 - shows a schematic view of the instrument for fluid filling with inserted dilatation probe for implantation in body cavities, according to the invention.
Figure 2 - shows a view of the instrument for fluid filling, as shown in Figure 1, in a vertical cross section.
Figure 3 - shows the dilatation probe for implantation in body cavities, as shown in Figure 1, in partial cross section
Figure 3a - shows the dilatation probe for implantation, as shown in Figure 1, in cross section along the A-A line
Figure 4 - shows the dilatation probe as a variant for the dilatation of the gullet
Figure 4a - shows the dilatation probe, as shown in Figure 1, in cross section along the B-B line

### The detailed description of the invention

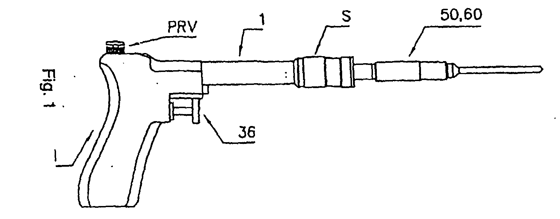

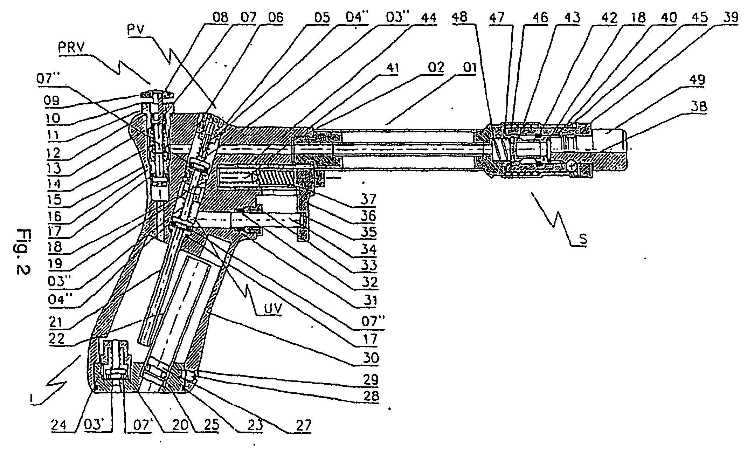

The invention, Figure 1, refers to the instrument I for fluid filling, and the dilatation probe 50, 60 for implantation in body cavities inserted wherein. The instrument I, Figure 2, basically consists of the: handle 30, filling valve 24, relief-adjusting valve PRV, intake valve UV, thrust valve PV, cylindrical extension 1 and a coupling S for the attachment of the dilating probe 50, 60. A "fluid" needed for the operation of the instrument assumes distilled water as the optimal solution, although other medically acceptable fluids could be used.

The handle 30 of the instrument I, Figure 2, is enclosed by a cover 29 at the base, which is sealed with the "O" ring 23 and secured from falling out by means of the bolt 28. On the inner side, on the cover 29, and in the handle cavity 30, the filling valve 24, used for pouring in of distilled water necessary for the operation of the instrument is attached. The filling valve 24 is sealed with the "O" ring 7', mounted on the valve cap 20, under the spring 3'.

Near the filling valve, inside the handle cavity 30, the cylinder of a variable volume 22 is located. Inside a cylinder 22, which is attached to a handle cover 29 by means of a threaded joint, and sealed with the "O" ring 25, is a movable piston 27 sealed with the "O" ring 26.
In the handle body 30, on the left upper part, the relief-adjusting valve PRV is mounted and is in contact with the handle cavity 30 with it's base. The relief-adjusting valve PRV consists of the piston 8 with the knob 9 sealed in a valve body 11 by means of the "O" ring 7. The valve is closed on the outside with the cap 10, while the valve body 11 is sealed in the handle 30 with the "O" ring 12 and the "O" ring 16. At the bottom of the valve PRV there is the adjusting nut 13 bolted on the valve shutter 15. The adjusting nut 13 applies a constant pressure to the adjusting spring 14, thereby pressing the shutter 15 against the surface of the "O" ring 17.

The intake valve UV, is located along the axis of the handle body 30, and is in connection, through the intake tube 21, which is sealed in the handle 30 by means of the "O" ring 17, with the cavity in the handle 30, and the piston 34 of the trigger 36. The intake valve consists of the "O" ring 7" and the valve shutter 4" pressed by the spring 3". The "O" ring 7" and the divider 19, form an area sealed from the handle 30 by means of the "O" ring 18. At the upper part and along the intake valve UV axis the thrust valve PV is located. It is in connection with the central canal, which is located in the upper part and goes through the handle 30 and the cylindrical extension 1. The thrust valve PV consists of the "O" ring 7", valve shutter 4" pressed by the spring 3" and guided in the cover 5, which is sealed from the handle 30 by means of the "O" ring 6.

In the front part of the handle 30 and beneath the extension 1 there is a trigger 36 sliding through the bushing 44 and returned by the spring 37 resting on the spring guide 41. The piston 34 is connected to the lower part of the trigger 36, through the plug 33 and "O" ring 32 pressed against the retainer 31, by means of the pin 35.

The handle 30 of the instrument I is connected to the coupling S, for the attachment of the dilating probe 50, 60, by the tubular extension 1, while the extension 1 is connected to the handle with the seal in between. Inside and coaxially with the extension there is a tube, which directly connects the cannel in the handle 30 with the coupling S.

The coupling S, for the attachment of the dilating probe 50, 60, which could be named fast coupling because of the short period of time needed for the probe to be inserted within, consists of the body 39, with the lower half 43 and upper half 40 placed inside. Located inside the body 39 there is the extension 49, which seals the intake over the seal 18, thus enabling quality filling of the cannel in the instrument I. The coupling body 39 is sealed in the extension 1 with the "O" ring 47. At the outside of the circumference of the body 39, the spring 46 is placed, as well as the coupling sleeve 45. The "O" ring 42 is placed between the halves 40, 43, and the body 39, while the balls 38 are placed at the inlet of the body 39.

The operation of the instrument I runs as follows: the distilled water, necessary for the operation of the instrument, is poured in the handle 30 of the instrument I, enclosed by a cover 29 at the base, which is sealed with the "O" ring 23 and secured from falling out by means of the bolt 28, through the filling valve 24, sealed with the "O" ring 7', mounted on a valve cap 20, under the spring 3'. While pouring the water in the body 39 of the fast coupling S, the extension 49 is attached, thus ensuring the quality filling of the cannel in the instrument. The filling is done with the syringe for single use, until the water appears at the inlet of the extension 49. When the filling is completed, the dilatation probe 50, 60 is attached to the coupling body 39, which is sealed in the extension 1 with the "O" ring 47. The probe, leaning against the "O" ring 18, pushes the upper half 40, the spring 48 and the lover half 43 until the upper half 40 leans against the "O" ring 42, enabling the balls 38 to snap into the cannel of the dilatation probe 50, 60. When this is done, the spring 46 of the sleeve 45 pushes the sleeve thus locking and sealing the dilatation probe 50, 60 in the coupling body 39. When the instrument I is filled up and the dilatation probe 50, 60 inserted in the instrument consecutively pressing the trigger 36, sliding through the bushing 44 and returned by the spring 37 resting on the spring guide 41, with a finger, the movement of the piston 34, through the plug 33 and "O" ring 32, by means of the pin 35, is exerted. The "O" ring 32 is pressed against the retainer 31, thus producing pressure and vacuum in a cannel in which it slides through. In a vacuum producing cycle, through the intake tube 21, which is sealed in the handle by means of the "O" ring 17, and the intake valve UV, the water is taken out of the cavity - space in the handle 30. The water is held in space formed by the "O" ring 7" and the divider 19, sealed from the handle 30 by means of the "O" ring 18. The movement of the variable volume piston 27, sealed with the "O" ring 26 in the variable volume cylinder 22, compensates the volume of the fluid taken, by means of reduction of the volume of the space in the handle 30 for the amount of the water taken. The variable volume cylinder 22, which is attached to a cover 29 of the handle 30 by means of a threaded joint, is sealed with the "O" ring 25. In a pressure producing cycle, the piston 34 pushes the water through the thrust valve PV. The water passes through the extension 1, lower half 43 and upper half 40 in the dilatation probe 50, 60. With the multiple cycle repetition, the pressure in the dilatation probe 50, 60 rises to the value adjusted with the relief-adjusting valve PRV. When the preset pressure value is reached, due to the overcome of the force in the regulating spring 14, the shutter 15 of the valve PRV detaches from the "O" ring 17, thus letting the water flow back in the handle 30, maintaining the constant pressure in the dilatation probe 50, 60, within the given values. In a moment when a pressure has reached the preset value, pressing the valve knob 9 exerts the piston 8, of the knob 9, movement, which pushes the shutter 15 of the valve and opens the valve, thus letting the water from the dilatation probe 50, 60 flow back to the handle 30. The variable volume piston 27 moves inside the variable volume cylinder 25, to enable the volume increase in the handle 30, absorbing the fluid from the dilatation probe 50, 60, thus preventing the pressure increase in the handle. During the sterilization process, the tip of the instrument is, for the protection of the instrument I, protected from damage by means of the protective cap, ensuring the dilation of the water found in the cannels.

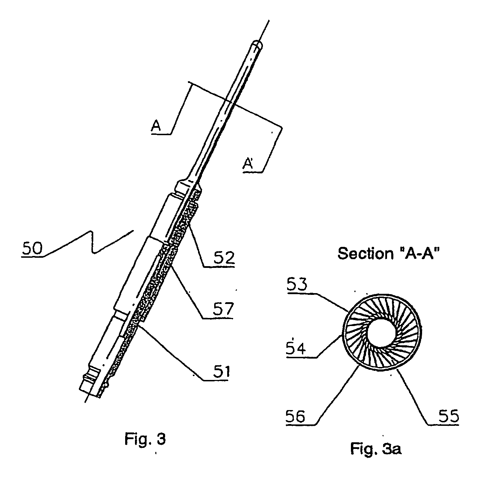

The dilatation probe 50, as shown in the Figure 3 and 3a, consists of the probe fitting 51, which is to be inserted in the coupling S of the instrument I shown in the Figure 2. Inside the probe itself, and in the extension of the fitting 51, there is a space enclosed by the inner elastic diaphragm 55 and the armored fabric 56 made of polyethylene with directed molecular chains wrapped in the paper wrapping 54. All this is shrouded with the probe cover 52, which presses the end of the inner elastic diaphragm 55 against the cannel in the probe fitting 51. Outer elastic diaphragm 53, which holds the injected fluid, and is attached to the cannel of the probe cover 52, is placed over the whole package consisting of the elements 52, 54, 55 and 56. The performance of the dilatation probe 50 is accomplished by insertion of the probe fitting 51 in the coupling S of the instrument I, with the pressurized liquid delivered through the opening 57 in the space enclosed by the inner elastic diaphragm 55. The diaphragm dilates, under the pressure, until the dimensions, previously defined by the armored fabric 56 wrapped in a paper wrapping 54, are attained. This causes the dilatation of the human cavity in which the probe is implanted, while the sealing, needed for the making of the watertight space suitable to be put under pressure, is accomplished with the probe cover 52, which presses the end of the inner elastic diaphragm 5 against the cannel in the probe fitting 51. In case the rupture of the inner elastic diaphragm 55 occurs, the outer elastic diaphragm 53, attached to the cannel of the probe cover 52, which holds the injected fluid, is positioned over the whole package. Since the probe is an element for single usage only, the rigidity of the package implanted in a body cavity is accomplished by means of the paper wrapping which tears with the dilation of the inner elastic diaphragm, thus enabling the dilation of the package and preventing the reusing of the probe, which looses the necessary rigidity for implanting in a human cavity after the tearing of the paper wrapping 54. The probe 50, as an element for single use, is shipped for an intervention packed and sterilized with a cobalt bomb.

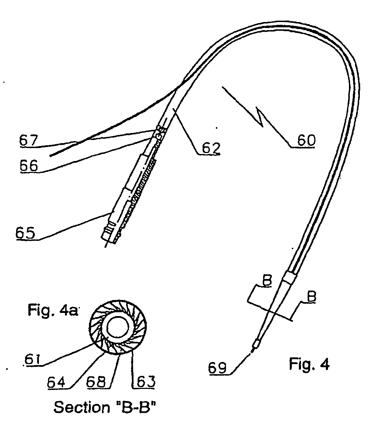

The dilatation probe 60, as a production variant, shown on Figure 4 and 4a, is aimed for gullet dilatation, and consists of the fitting 65 for the instrument I, with the ball 66 and the buffer 67 of the nonreturn valve placed at the inlet. Along the hose 62 for the pressurized fluid, wrapped with the outer latex diaphragm 63, armored fabric 68 and the inner latex diaphragm 64, there is a feeding hose 61 placed with the feeding opening 69. The performance of the probe is achieved by implanting the dilatation probe through a nose until the dilating part reaches the place where the stenosys is. When this is done, the pumping of the fluid with the instrument I begins, causing the dilatation. The dilatation is performed through the nonreturn valve until the diameter requested by the surgeon is achieved. After the dilatation is finished, the instrument is detached and the probe stays at the position for the time needed for the tissue accommodation. The nonretum valve maintains the pressure in the probe. After that, the instrument is attached again for further dilatation. This procedure is repeated until the end of dilatation. When the dilatation is finished, the hose for the pressurized fluid is cut and the probe is pulled out of the gullet. The probe is made of thermolabile material and is for a single usage only, so the sterilization is performed in a packed form with a cobalt bomb.

### Industrial and other applications of the invention

The invention application originates in an obvious manner from the previous text and included drawings, so further description is not necessary. The inventor checked all the functional and design characteristics of the invention at the prototype.

## Claims

1. An instrument for fluid injection and a disposable dilatation probe (**50, 60**) for implantation in body cavities comprising a cylindrical extension (**1**) a cover (**29**) for the handle (**30**) of the instrument (**I**), with a filling valve (**24**) and a variable volume cylinder (**22**) attached to it; where inside the handle (**30**) are: a relief-adjusting valve (**PRV**), an intake valve (**UV**) connected to a trigger (**36**) by means of a piston (**34**), and a thrust valve (**PV**); with a coupling (**S**) attached to the free end of the cylindrical extension (**1**) and a coaxial tube inside it for a direct link with the channel inside the handle (**30**) for the attachment of the disposable dilating probe (**50, 60**), **characterized in that** the relief-adjusting valve (**PRV**) is mounted in the handle body (**30**), opposite to the cylindrical extension (**1**), and is in contact with the handle (**30**) cavity with it's base and **in that** said relief-adjusting valve (**PRV**) comprises a piston (**8**) with a knob (**9**) on the top, sealed inside a valve body (**11**) by means of an "**O**" ring (**7**); and said valve (**PRV**) is closed on the outside with a cap (**10**), while the valve body (**11**) is sealed in the handle (**30**) with two "**O**" rings (**12, 16**); and at the bottom of the valve (**PRV**) is an adjusting nut (**13**) bolted on a valve shutter (**15**) said adjusting nut (**13**) applying a constant pressure to an adjusting spring (**14**), thereby pressing the shutter (**15**) against the surface of an "**O**" ring (**17**).

2. The instrument for fluid injection and the disposable dilatation probes (**50, 60**) for implantation in body cavities, of claim 1, further **characterized with** that the intake valve (**UV**) located along the axis of the handle body (**30**) is in connection, through an intake tube (**21**) sealed in the handle (**30**) by means of the "**O**" ring (**17**), with the cavity in the handle (**30**), and the piston (**34**) of the trigger (**36**), where said intake valve (**UV**) consists of an."**O**" ring (**7**") and a valve shutter (**4"**) pressed by a spring (**3"**) where the "**O**" ring (**7"**) and a divider (**19**) form an area sealed from the handle (**30**) by means of an "**O**" ring (**18**), and where the thrust valve (**PV**) is located at the upper part and along the intake valve (**UV**) axis, where said thrust valve (**PV**) is in connection with the central channel, which is located in the upper part and goes through the handle (**30**) and the cylindrical extension (**1**) and said thrust valve (**PV**) consists of the "**O**" ring (**7"**), valve shutter (**4"**) pressed by the spring (**3"**) and guided in the cover (**5**), which is sealed from the handle (**30**) by means of an **"O"** ring (**6**).

3. The instrument for fluid injection and the disposable dilatation probes **50**, **60** for implantation in body cavities, of claims 1 and 2, further **characterized with** that the handle (**30**) of the instrument **(I)** is connected to the coupling (**S**), for the attachment of the dilating probe (**50**, **60**), by the tubular extension (**1**), inside of which and coaxially with the extension (**1**) is a tube, which directly connects the channel in the handle (**30**) with the coupling (**S**) while the extension (**1**) is connected to the handle (**30**) with the seal in between, and where said coupling (**S**) consists of a body (**39**), with the lower half (**43**) and upper half (**40**) placed inside, where inside the body (**39**) there is located the extension (**49**), which seals the intake over the seal (**18**), thus enabling quality filling of the channel in the instrument (**I**) and where said coupling body (**39**) is sealed in the extension (**1**) with the **"O"** ring (**47**), and where at the outside of the circumference of the body (**39**) is placed a spring (**46**) and a coupling sleeve (**45**) and an **"O"** ring (**42**) is placed between the halves (**40, 43**) and the body (**39**), while balls (**38**) are placed at the inlet of the body (**39**).

4. The instrument for fluid injection and the disposable dilatation probes **50**, **60** for implantation in body cavities, of claims 1, 2 and 3, further **characterized with** that the disposable dilatation probe (**50**) consists of a probe fitting (**51**), which is to be inserted in the coupling (**S**) of the instrument (**I**) where inside the probe (**50**), and in the extension of the fitting (**51**) is a space enclosed by an inner elastic diaphragm (**55**) and an armored fabric (**56**) made of polyethylene with directed molecular chains wrapped in a paper wrapping (**54**) where all this is shrouded with a probe cover (**52**), which presses the end of the inner elastic diaphragm (**55**) against the channel in the probe fitting (**51**) and where outer elastic diaphragm (**53**), which holds the injected fluid, and is attached to the channel of the probe cover (**52**), is placed over the whole package consisting of the elements (**52**, **54**, **55** and **56**).

5. The instrument for fluid injection and the disposable dilatation probes **50**, **60** for implantation in body cavities, of claims 1, 2 and 3, further **characterized with** that the dilatation probe (**60**) aimed for gullet dilatation consists of a fitting (**65**) for the instrument (**I**), with a ball (**66**) and a buffer (**67**) of the non-return valve placed at the inlet of the fitting (**65**), and that along a hose (**62**) for the pressurized fluid, wrapped with an outer latex diaphragm (**63**), armored fabric (**68**) and an inner latex diaphragm (**64**), there is a feeding hose (**61**) placed with an feeding opening (**69**)

## Patentansprüche

1. Instrument zum Einspritzen eines Fluids und Erweiterungssonde (50, 60) zur einmaligen Verwendung, die in Hohlräume im Körper einzusetzen ist, mit einer zylindrischen Verlängerung (1), einer Abdeckung (29) für den Griff (30) des Instruments (I), wobei ein Auffüllventil (24) und ein Zylinder (22) mit variablem Volumen daran befestigt sind; wobei sich im Inneren des Griffs (30) Folgendes befindet: ein Ablass-Einstellventil (PRV), ein Einlassventil (UV), das mittels eines Kolbens (34) mit einem Auslöser (36) verbunden ist, und ein Druckventil (PV); mit einem Verbindungsstück (S), das am freien Ende der zylindrischen Verlängerung (1) befestigt ist und einer koaxialen Röhre im Inneren zur direkten Verbindung mit dem Kanal im Inneren des Griffs (30), um die Erweiterungssonde (50, 60) zur einmaligen Verwendung daran zu befestigen, **dadurch gekennzeichnet, dass** das Ablass-Einstellventil (PRV) im Griffkörper (30) gegenüber der zylindrischen Verlängerung (1) angebracht und mit seiner Basis mit dem Hohlraum des Griffs (30) in Kontakt ist, und dass das Ablass-Einstellventil (PRV) einen Kolben (8) mit einem Knopf (9) an der Oberseite aufweist und mittels eines O-Rings (7) im Inneren eines Ventilkörpers (11) abgedichtet ist; wobei das Ventil (PRV) an der Außenseite mit einer Kappe (10) verschlossen ist, während der Ventilkörper (11) in dem Griff (30) mit zwei O-Ringen (12, 16) abgedichtet ist; und wobei sich an der Unterseite des Ventils (PRV) eine Einstellmutter (13) befindet, die an einer Ventilklappe (15) festgeschraubt bzw. mit Bolzen befestigt ist, wobei die Einstellmutter (13) einen konstanten Druck auf eine Einstellfeder (14) ausübt, wodurch die Klappe (15) gegen die Oberfläche eines O-Rings (17) gedrückt wird.

2. Instrument zum Einspritzen eines Fluids und Erweiterungssonden (50, 60) nach Anspruch 1 zur einmaligen Verwendung, die in Hohlräume im Körper einzusetzen sind, außerdem **dadurch gekennzeichnet, dass** das Einlassventil (UV), das sich auf der Achse des Griffkörpers (30) befindet, über eine Einlassröhre (21), die in dem Griff (30) durch den O-Ring (17) abgedichtet ist, mit dem Hohlraum in dem Griff (30), aber auch mit dem Kolben (34) des Auslösers (36) in Verbindung steht, wobei das Einlassventil (UV) aus einem O-Ring (7") und einer von einer Feder (3") beaufschlagten Ventilklappe (4") besteht, wobei der O-Ring (7'') und ein Trennelement (19) einen Bereich bilden, der durch einen O-Ring (18) von dem Griff (30) abgedichtet ist, und wobei das Druckventil (PV) sich im oberen Bereich und auf der Achse des Einlassventils (UV) befindet und das Druckventil (PV) mit dem Mittelkanal in Verbindung steht, der sich im oberen Bereich befindet und durch den Griff (30) und die zylindrische Verlängerung (1) läuft, und wobei das Druckventil (PV) aus dem O-Ring (7'') und der Ventilklappe (4'') besteht, die durch die Feder (3") beaufschlagt und in der Abdeckung (5) geführt ist, die durch einen O-Ring (6) von dem Griff (30) abgedichtet ist.

3. Instrument zum Einspritzen eines Fluids und Erweiterungssonden (50, 60) nach Anspruch 1 und 2 zur einmaligen Verwendung, die in Hohlräume im Körper einzusetzen sind, außerdem **dadurch gekennzeichnet, dass** der Griff (30) des Instruments (I) durch die röhrenförmige Verlängerung (1) mit dem Verbindungsstück (S) zum Anbringen der Erweiterungssonde (50, 60) verbunden ist, wobei sich in der Verlängerung (1) und koaxial zu dieser eine Röhre befindet, die den Kanal in dem Griff (30) direkt mit dem Verbindungsstück (S) verbindet, während die Verlängerung (1) mit dem Griff (30) mit der dazwischen liegenden Dichtung verbunden ist, wobei das Verbindungsstück (S) aus einem Körper (39) besteht, mit der unteren Hälfte (43) und der oberen Hälfte (40) im Inneren, wobei sich in dem Körper (39) die Verlängerung (49) befindet, die den Einlass über der Dichtung (18) abdichtet, wodurch ein gutes Füllen des Kanals in dem Instrument (I) ermöglicht wird, wobei der Körper (39) des Verbindungsstücks in der Verlängerung (1) mit dem O-Ring (47) abgedichtet ist, und wobei an der Außenseite des Umfangs des Körpers (39) eine Feder (46) und eine Verbindungsmuffe (45) platziert sind und ein O-Ring (42) zwischen den Hälften (40, 43) und dem Körper (39) angeordnet ist, während am Einlass des Körpers (39) Kugeln (38) platziert sind.

4. Instrument zum Einspritzen eines Fluids und Erweiterungssonden (50, 60) nach Anspruch 1, 2 und 3 zur einmaligen Verwendung, die in Hohlräume im Körper einzusetzen sind, außerdem **dadurch gekennzeichnet, dass** die Erweiterungssonde (50) zur einmaligen Verwendung ein Sonden-Anschlussstück (51) aufweist, das in das Verbindungsstück (S) des Instruments (I) einzusetzen ist, wobei im Inneren der Sonde (50) und in der Verlängerung des Anschlussstücks (51) ein Raum vorhanden ist, der von einer inneren elastischen Membran (55) und einem verstärkten Stoff (56) aus Polyethylen mit ausgerichteten Molekülketten umschlossen ist, umhüllt von einer Papierhülle (54), wobei all dies von einer Sondenabdeckung (52) abgedeckt ist, die das Ende der inneren elastischen Membran (55) gegen den Kanal in dem Sonden-Anschlussstück (51) drückt, und wobei die äußere elastische Membran (53), die das eingespritzte Fluid hält und am Kanal der Sonden-Abdeckung (52) befestigt ist, über der gesamten Anordnung, die aus den Elementen (52, 54, 55 und 56) besteht, platziert ist.

5. Instrument zum Einspritzen eines Fluids und Erweiterungssonden (50, 60) nach Anspruch 1, 2 und 3 zur einmaligen Verwendung, die in Hohlräume im Körper einzusetzen sind, außerdem **dadurch gekennzeichnet, dass** die Erweiterungssonde (60), die der Speiseröhrenerweiterung dient, ein Anschlussstück (65) für das Instrument (I) aufweist, wobei eine Kugel (66) und ein Puffer (67) des Rückschlagventils am Einlass des Anschlussstücks (65) platziert sind, und dass entlang eines Schlauchs (62) für das unter Druck gesetzte Fluid, umhüllt mit einer äußeren Latexmembran (63), einem verstärkten Stoff (68) und einer inneren Latexmembran (64), ein Zuführschlauch (61) mit einer Zuführöffnung (69) vorgesehen ist.

## Revendications

1. Instrument d'injection de fluide et sonde de dilatation jetable (50, 60) pour implantation dans des cavités corporelles, comportant une extension cylindrique (1), un recouvrement (29) pour la poignée (30) de l'instrument (I), ayant une soupape de remplissage (24) et un cylindre à volume variable (22) fixés dessus, où à l'intérieur de la poignée (30) se trouvent : une soupape de régulation (PRV), une soupape d'admission (UV) connectée à un déclencheur (36) par l'intermédiaire d'un piston (34), et une soupape de poussée (PV), un raccord (S) étant relié à l'extrémité libre de l'extension cylindrique (1) et à un tube coaxial situé à l'intérieur de celle-ci pour une liaison directe avec le canal situé à l'intérieur de la poignée (30) pour la fixation de la sonde de dilatation jetable (50, 60), **caractérisés en ce que** la soupape de régulation (PRV) est montée dans le corps de poignée (30), opposée à l'extension cylindrique (1), et est en contact avec la cavité de la poignée (30) avec sa base, et **en ce que** ladite soupape de régulation (PRV) comporte un piston (8) ayant un bouton (9) sur la partie supérieure, rendu étanche à l'intérieur d'un corps de soupape (11) par l'intermédiaire d'un joint torique (7), et ladite soupape (PRV) est fermée sur l'extérieur à l'aide d'un couvercle (10), tandis que le corps de soupape (11) est rendu étanche dans la poignée (30) à l'aide de joints toriques (12, 16), et au niveau de la partie inférieure de la soupape (PRV) se trouve un écrou de régulation (13) boulonné sur un obturateur de soupape (15), ledit écrou de régulation (13) appliquant une pression constante sur un ressort de régulation (14), en comprimant ainsi l'obturateur (15) contre la surface d'un joint torique (17).

2. Instrument d'injection de fluide et sondes de dilatation jetables (50, 60) pour implantation dans des cavités corporelles selon la revendication 1, **caractérisés en outre en ce que** la soupape d'admission (UV) positionnée le long de l'axe du corps de poignée (30) est connectée, à travers un tube d'admission (21) rendu étanche dans la poignée (30) par l'intermédiaire du joint torique (17), à la cavité située dans la poignée (30), et le piston (34) du déclencheur (36), où ladite soupape d'admission (UV) est constituée d'un joint torique (7") et d'un obturateur de soupape (4") comprimé par un ressort (3"), où le joint torique (7") et un diviseur (19) forment une zone rendue étanche vis-à-vis de la poignée (30) par l'intermédiaire d'un joint torique (18), et où la soupape de poussée (PV) est positionnée au niveau de la partie supérieure et le long de l'axe de la soupape d'admission (UV), où ladite soupape de poussée (UV) est connectée au canal central, qui est positionné dans la partie supérieure et qui passe à travers la poignée (30) et l'extension cylindrique (1), et où ladite soupape de poussée (PV) est constituée du joint torique (7"), de l'obturateur de soupape (4") comprimé par le ressort (3") et guidé dans le recouvrement (5), qui est rendu étanche vis-à-vis de la poignée (30) par l'intermédiaire d'un joint torique (6).

3. Instrument d'injection de fluide et sondes de dilatation jetables (50, 60) pour implantation dans des cavités corporelles selon les revendications 1 et 2, **caractérisés en outre en ce que** la poignée (30) de l'instrument (I) est connectée au raccord (S) pour la fixation de la sonde de dilatation (50, 60), par l'intermédiaire de l'extension tubulaire (1), à l'intérieur de laquelle se trouve un tube, qui est coaxial à l'extension (1), qui connecte directement le canal situé dans la poignée (30) au raccord (S) tandis que l'extension (1) est connectée à la poignée (30) en ayant le joint d'étanchéité entre ceux-ci, et où ledit raccord (S) est constitué d'un corps (39), la moitié inférieure (43) et la moitié supérieure (40) étant placées à l'intérieur, où à l'intérieur du corps (39) est positionnée l'extension (49), qui rend étanche l'admission sur le joint d'étanchéité (18), en permettant ainsi un remplissage de qualité du canal situé dans l'instrument (I), et où ledit corps de raccord (39) est rendu étanche dans l'extension (1) avec le joint torique (47), et où au niveau de l'extérieur de la circonférence du corps (39) sont placés un ressort (46) et un manchon de raccord (45), et un joint torique (42) est positionné entre les moitiés (40, 43) et le corps (39), tandis que des billes (38) sont placées au niveau de l'entrée du corps (39).

4. Instrument d'injection de fluide et sondes de dilatation jetables (50, 60) pour implantation dans des cavités corporelles selon les revendications 1, 2 et 3, **caractérisés en outre en ce que** la sonde de dilatation jetable (50) est constituée d'un raccord de sonde (51), qui doit être inséré dans le raccord (S) de l'instrument (I), où à l'intérieur de la sonde (50) et dans le prolongement du raccord (51) se trouve un espace délimité par une membrane élastique intérieure (55) et un tissu à armure (56) en polyéthylène ayant des chaînes moléculaires dirigées enveloppées dans un enveloppement de papier (54) où la totalité est protégée par un recouvrement de sonde (52), qui comprime l'extrémité de la membrane élastique intérieure (55) contre le canal situé dans le raccord de sonde (51), et où la membrane élastique extérieure (53), qui maintient le fluide injecté, et qui est reliée au canal du recouvrement de sonde (52), est placée sur le conditionnement total constitué des éléments (52, 54, 55 et 56).

5. Instrument d'injection de fluide et les sondes de dilatation jetables (50, 60) pour implantation dans des cavités corporelles selon les revendications 1, 2 et 3, **caractérisés en outre en ce que** la sonde de dilatation jetable (60) a pour but une dilatation de gosier est constituée d'un raccord (65) pour l'instrument (I), une bille (66) et un tampon (67) du clapet anti-retour étant placés au niveau de l'entrée du raccord (65), et **en ce que** le long d'un tuyau (62) pour le fluide mis sous pression, enveloppé avec une membrane en latex extérieure (63), un tissu à armure (68) et une membrane en latex intérieure (64), se trouve un tuyau d'alimentation (61) placé avec l'ouverture d'alimentation (69).
